# EUROPÄISCHE PATENTSCHRIFT

(11) **EP 4 197 563 B1**
(45) Veröffentlichungstag und Bekanntmachung des Hinweises auf die Patenterteilung: **20.03.2024**
(21) Anmeldenummer: 21215071.8
(22) Anmeldetag: 16.12.2021
(51) Int. Cl.: A61L 2/00, A61L 2/10, A61L 2/24

(54) **VERFAHREN ZUM BETREIBEN EINER MINDESTENS EINE UV-LED UMFASSENDEN DESINFEKTIONSEINRICHTUNG UND DESINFEKTIONSEINRICHTUNG**
DISINFECTION DEVICE AND METHOD FOR OPERATING A DISINFECTING DEVICE COMPRISING AT LEAST ONE UV LED
PROCÉDÉ DE FONCTIONNEMENT D'UN DISPOSITIF DE DÉSINFECTION COMPRENANT AU MOINS UNE DEL UV ET DISPOSITIF DE DÉSINFECTION

(43) Veröffentlichungstag der Anmeldung: 21.06.2023
(73) Patentinhaber: Lumitech Patentverwertung GmbH, 8380 Jennersdorf (AT)
(72) Erfinder: HOSCHOPF, Hans-Christian, 8380 Jennersdorf (AT); TASCH, Stefan, 8380 Jennersdorf (AT)
(74) Vertreter: Feucker, Max Martin

(56) Entgegenhaltungen:
- CN-A- 111 658 796
- KR-A- 20150 025 660

## Beschreibung

Die vorliegende Erfindung betrifft eine Desinfektionseinrichtung mit mindestens einer ultraviolettes Licht emittierenden UV-LED und ein Verfahren zum Betreiben einer solchen LED. Die Desinfektionseinrichtung umfasst also die ultraviolettes Licht emittierende UV-LED und eine Steuereinheit, die insbesondere zur Durchführung des Verfahrens eingerichtet ist.

Ultraviolettes Licht emittierende Desinfektionseinrichtungen sind als solche bekannt. Die von dem ultravioletten Licht bestrahlten Oberflächen werden desinfiziert, wozu aber eine gewisse Bestrahlungsstärke erforderlich ist, bei der davon ausgegangen werden kann, dass die Viren/Bakterien auf der Oberfläche durch die ultraviolette Strahlung inaktiviert wurden. Es ist also je nach Strahlungsquelle sicherzustellen, dass die zu desinfizierende Oberfläche genügend lange/stark bestrahlt wird. So könnte beispielsweise eine entsprechende Desinfektionseinrichtung manuell für einen vorgegebenen Zeitraum täglich ein- und ausgeschaltet werden. Die manuelle Betätigung ist aber mit einem gewissen Aufwand verbunden. Aus KR 2015 0025660 A ist eine mobile Desinfektionseinrichtung bekannt, die einen Zeitgeber umfasst und damit einen Gegenstand gemäß dem Oberbegriff des Anspruch 12 darstellt.

Hinzu kommt, dass bei der Verwendung von ultraviolettes Licht emittierenden LEDs die Strahlungsleistung der LEDs bei gleichem Versorgungsstrom mit zunehmender Betriebsdauer abnimmt.

Aufgabe der vorliegenden Erfindung ist es daher, eine Desinfektionseinrichtung anzugeben, mit der eine ausreichende Desinfektion der bestrahlten Oberfläche sichergestellt ist.

Gelöst wird die Aufgabe durch eine Desinfektionseinrichtung und ein Verfahren mit den Merkmalen des jeweiligen unabhängigen Anspruchs. Vorteilhafte Weiterbildungen der Desinfektionseinrichtung und des Verfahrens sind in den abhängigen Ansprüchen und in der Beschreibung angegeben, wobei einzelne Merkmale der vorteilhaften Weiterbildungen in technisch sinnvoller Weise miteinander kombinierbar sind.

Die Aufgabe wird insbesondere durch ein Verfahren zum Betreiben einer mindestens eine UV-LED umfassenden Desinfektionseinrichtung gelöst, welches zumindest die folgenden Schritte umfasst:
- Bestromen der mindestens einen UV-LED mit einem Versorgungsstrom,
- Bereitstellen eines Zeitsignals,
- Betreiben der Desinfektionseinrichtung unter Verwendung des Zeitsignals.

Entsprechend wird die Aufgabe durch eine Desinfektionseinrichtung gelöst, die mindestens eine ultraviolettes Licht emittierende UV-LED und einen Zeitgeber umfasst.

Die Desinfektionseinrichtung umfasst bevorzugt eine Steuereinheit. Die Steuereinheit der Desinfektionseinrichtung kann ein Vorschaltgerät zur Versorgung des LED-Moduls mit Gleichstrom umfassen, wobei das Vorschaltgerät mittels eines entsprechenden Treibers steuer- oder regelbar ausgeführt sein kann, um die Versorgungsstromhöhe einzustellen. Das Vorschaltgerät ist hierzu in geeigneter Weise mit der Steuereinheit beziehungsweise mit einem Prozessor der Steuereinheit auf geeignete Weise (drahtlos, drahtgebunden) verbunden, so dass entweder digitale oder analoge Signale übertragen werden können.

Unter Verwendung des Zeitgebers kann beispielsweise der Einschaltzeitpunkt, der Ausschaltzeitpunkt und/oder die Einschaltdauer erfasst und/oder vorgegeben werden. Die Zeitsignale des Zeitgebers können über einen RTC (Real Time Clock)-Baustein, DCF77-Signalerfassung, GPS-Signalerfassung, LAN, WLAN, WIFI-Anbindung oder Ähnliches generiert werden. Für den Zeitgeber kann zur Überbrückung einer Stromunterbrechung eine Batterie vorgesehen sein. Zudem kann die Bestromungsdauer, mit der die UV-LED mit Versorgungsstrom bestromt wird, erfasst werden. Die Bestromungsdauer und gegebenenfalls die Versorgungsstromhöhe können von der Steuereinheit erfasst werden.

Es kann insbesondere vorgesehen sein, dass die Desinfektionseinrichtung und bevorzugt deren Steuereinheit so eingerichtet ist, dass die mindestens eine UV-LED unter Verwendung der von dem Zeitgeber bereitgestellten Zeitsignale in vorgebbaren Intervallen und/oder Zeiten automatisch mit einem Versorgungsstrom bestromt wird. Beispielsweise könnte (beispielsweise durch Hinterlegung entsprechender Daten in der Steuereinheit) vorgegeben werden, dass die UV-LED in einem gewissen Zeitfenster automatisch bestromt wird.

Soweit bekannt ist, wann der Raum, in dem die Desinfektionseinrichtung betrieben wird, gereinigt wird, kann in der Steuereinheit hinterlegt sein, dass die Bestromung in diesem Zeitraum unter Verwendung der von dem Zeitgeber bereitgestellten Zeitsignale unterbrochen wird.

Zudem kann in der Steuereinheit hinterlegt sein, wann eine Wartung beziehungsweise Inspektion zu erfolgen hat. In diesem Fall ist die Steuereinheit dazu eingerichtet, unter Verwendung eines von dem Zeitgeber bereitgestellten Zeitsignals ein entsprechendes Signal zu senden.

Es kann auch vorgesehen sein, dass die Bestromung der mindestens einen UV-LED manuell gestartet wird, wobei eine Betriebsdauer vorgegeben wird, nach der die Bestromung unter Verwendung eines von dem Zeitgeber bereitgestellten Zeitsignals gestoppt wird.

Durch das Bereitstellen eines Zeitsignals beziehungsweise durch das Vorsehen eines Zeitgebers kann die Desinfektionseinrichtung zumindest teilweise automatisch und damit weitestgehend autark betrieben werden, wobei durch die Vorgabe entsprechender Parameter (beispielsweise Bestromungsdauer) sichergestellt wird, dass auch eine ausreichende Desinfektion der bestrahlten Oberfläche stattfindet.

Um insbesondere bei einer automatischen Aktivierung der UV-LED zu verhindern, dass Lebewesen von dem emittierten Licht bestrahlt werden, kann die Desinfektionseinrichtung einen Sensor zur Erkennung eines Lebewesens und/oder einer Bewegung umfassen, wobei bei der Detektion eines Lebewesens und/oder einer Bewegung die Bestromung der UV-LED verhindert/unterbrochen wird. Sobald kein Lebewesen mehr mittels des Sensors detektiert wird, kann die Bestromung fortgesetzt werden. Auch die Unterbrechung der Bestromung kann von der Steuereinheit festgestellt werden. Der Sensor kann beispielsweise als Hochfrequenz-, Ultraschall-, Infrarot- oder Time of Flight-Sensor ausgebildet sein. Die Steuereinheit kann dabei eine Schaltung umfassen, mit der die Funktionsfähigkeit des Sensors überprüft werden kann.

Die Desinfektionseinrichtung kann auch ein Relais aufweisen, welches so verschaltet ist, dass bei der Detektion eines Lebewesens mittels des Sensors die Bestromung der UV-LED unterbrochen wird.

Erfindungsgemäß umfasst das Verfahren den folgenden Schritt:
- Hinterlegen von Daten in einer Steuereinheit der Desinfektionseinrichtung, wobei die Daten zumindest eine Größe des von der mindestens einen UV-LED emittierten Lichts in Abhängigkeit eines die mindestens eine UV-LED versorgenden Versorgungsstrom wiedergeben.

In diesem Fall kann bevorzugt vorgesehen sein, dass das Verfahren auch die folgenden Schritte umfasst:
- Erfassen einer Versorgungsstromhöhe des Versorgungsstroms,
- Erfassen einer Bestromungsdauer unter Verwendung des bereitgestellten Zeitsignals,
- Ermitteln einer Kennzahl des von der mindestens einen UV-LED emittierten Lichts auf Basis der erfassten Versorgungsstromhöhe und der erfassten Bestromungsdauer unter Rückgriff auf die hinterlegten Daten.

Dementsprechend ist erfindungsgemäß vorgesehen, dass in der Steuereinheit Daten hinterlegt sind, die zumindest eine Größe des von der mindestens einen UV-LED emittierten Lichts in Abhängigkeit eines die mindestens eine UV-LED versorgenden Versorgungsstroms wiedergeben.

Mit anderen Worten: Es wird insbesondere vorgeschlagen, dass in der Steuereinheit Daten (beispielsweise Kennlinien, Kennlinienfelder, Tabellen oder Ähnliches) hinterlegt sind, aus denen zumindest ein Parameter des emittierten UV-Lichts hervorgeht, der von der Höhe des Stroms abhängt, der durch die UV-LED fließt. In den Daten können (müssen aber nicht) weitere Abhängigkeiten der Parameter des emittierten Lichts von anderen Einflussgrößen hinterlegt sein. So kann der mindestens eine Parameter sowohl von dem Versorgungsstrom als auch von der Temperatur der UV-LED während der Bestrahlung abhängig sein. In diesem Fall würde auch eine Temperatur der UV-LED mit einem entsprechenden Sensor detektiert werden. Zudem kann der mindestens eine Parameter des von der UV-LED emittierten Lichts zusätzlich zu der Stromhöhe (und gegebenenfalls zusätzlich zu der Temperatur) von der zurückliegenden Betriebsdauer der UV-LED abhängen. In den Daten wäre also die Abhängigkeit des Parameters von dem Strom (und gegebenenfalls von der Temperatur) und von der zurückliegenden Betriebsdauer hinterlegt. Diese Daten werden vor der ersten regulären Inbetriebnahme der Desinfektionseinrichtung in der Steuereinheit hinterlegt. Die zu hinterlegenden Daten können beispielsweise durch Vermessung genau der in der Desinfektionseinrichtung eingesetzten UV-LED(s) oder einer UV-LED aus der gleichen Charge bestimmt worden sein. Sofern diese Daten für die UV-LEDs eines Herstellers nicht wesentlich fluktuieren, können auch die Daten eines Herstellers verwendet werden. Soweit die Daten auch die Degradation (also die Abnahme der Strahlungsleistung mit zunehmender Betriebsdauer bei gleichem Versorgungsstrom) wiedergeben, so kann diese Abhängigkeit von der zurückliegenden Betriebsdauer aus Erfahrungswerten für solche UV-LEDs extrapoliert werden. Die hinterlegten Parameter können beispielsweise der Strahlungsfluss, die (Peak)-Wellenlänge, die Strahlstärke, die spezifische Ausstrahlung, die Strahldichte oder die Strahlungsausbeute sein.

Die UV-LED kann mit einem konstanten Gleichstrom oder mit einem gepulsten Gleichstrom betrieben werden. Im ersten Fall ist die Abhängigkeit des Parameters des emittierten Lichts von der Höhe des konstanten Gleichstroms in den Daten hinterlegt. Im zweiten Fall ist die Abhängigkeit des Parameters des emittierten Lichts von der Pulshöhe, der Pulsbreite und/oder der Pulsfrequenz hinterlegt. Bevorzugt wird die UV-LED mit konstantem Gleichstrom betrieben, in welchem Fall ein entsprechender, konstanten Gleichstrom bereitstellender LED-Treiber vorgesehen ist.

Im Betrieb kann also die Höhe des konstanten Gleichstroms beziehungsweise die Pulshöhe, die Pulsdauer und/oder die Pulsfrequenz als Versorgungsstromhöhe erfasst werden. Hierzu kann die Steuereinheit die Versorgungsstromhöhe während des Betriebs feststellen und speichern.

Gegebenenfalls kann die Desinfektionseinrichtung auch einen Temperatursensor umfassen, mit dem die Temperatur zumindest einer UV-LED oder zumindest eines LED-Moduls gemessen und erfasst wird.

Im Folgenden ermittelt die Steuereinheit unter Rückgriff auf die hinterlegten Daten und unter Rückgriff auf die erfassten Daten eine Kennzahl, die eine physikalische Größe des emittierten Lichts repräsentiert. Es ist also nicht zwingend, dass der exakte nummerische Wert der physikalischen Größe bestimmt wird, so lange die Kennzahl diese Größe wiederspiegelt.

Die so ermittelte Kennzahl repräsentiert insbesondere die Strahlungsenergie des während des Bestromens emittierten Lichts. Sofern die Abstrahlcharakteristik der UV-LED und die Größe/Geometrie der bestrahlten Fläche (des Raumes) bekannt sind, so kann hierauf basierend auch die Bestrahlungsstärke und hierauf basierend wiederum der Desinfektionserfolg abgeschätzt werden.

In einer Ausführungsform kann vorgesehen sein, dass die ermittelte Kennzahl mit einer vorgegebenen Kennzahl verglichen wird und bei einer insbesondere in ihrer Höhe vorgebbaren Abweichung der ermittelten Kennzahl von der vorgegebenen Kennzahl ein Signal erzeugt wird. Dieses Signal kann beispielsweise ein akustisches oder optisches Signal sein. Das optische Signal kann beispielsweise durch eine Anzeige-LED oder ein Display oder Ähnliches direkt an der Desinfektionseinrichtung erzeugt werden. Die Anzeige-LED, das Display oder ein Lautsprecher zur Erzeugung eines akustischen Signals bilden somit eine Warneinheit aus. Es kann aber auch sein, dass das Signal über eine Kommunikationseinheit an eine übergeordnete (Kontroll-)Einheit und/oder direkt an ein Endgerät eines Benutzers übermittelt wird. Beispielsweise kann die Kommunikation über eine Internet-basierte Cloudanbindung erfolgen, worüber auch gezielt Benutzer über eine Abweichung informiert werden. Beispielsweise kann ein solches Signal erzeugt werden, wenn die ermittelte Bestrahlungsstärke (beziehungsweise die die Bestrahlungsstärke repräsentierende Kennzahl) nicht mit einer vorgegebenen Bestrahlungsstärke (Kennzahl) übereinstimmt. In diesem Fall könnte der Benutzer entscheiden, dass eine Desinfektion der bestrahlten Oberfläche mit anderen Methoden zu erfolgen hat.

Die Kommunikationseinheit kann zur Anbindung an einen PC, ein Smartphone, eine Gebäudesteuerung oder eine Zentrale, gegebenenfalls Cloud-basierte Überwachungsstelle, eingerichtet sein. Beispielsweise kann eine drahtgebundene Verbindung (DALI, KNX etc.) oder eine drahtlose (WLAN, ZigBee, Bluetooth) verwirklicht sein. Über dieses Kommunikationsmodul können auch Updates aufgespielt werden.

Die vorgegebene Kennzahl, mit der die ermittelte Kennzahl verglichen wird, kann einstellbar sein und insbesondere in Abhängigkeit einer Raumgröße und/oder einer Raumgeometrie (ggf. inklusive Möbeln) eines Raumes, in dem die Desinfektionseinrichtung betrieben wird, eingestellt werden. Für eine besonders nutzerfreundliche Handhabung kann vorgesehen sein, dass ein Benutzer den Abstand der Desinfektionseinrichtung zu der zu bestrahlenden Fläche eingibt und daraus die vorgegebene Kennzahl bestimmt wird.

Die Kennzahl kann beispielsweise am Ende einer jeden Bestromung ermittelt werden. Die Kennzahl kann aber auch nach einem insbesondere einstellbaren (Zeit)-Intervall für das zurückliegende Intervall bestimmt werden. Beispielsweise kann die Kennzahl täglich für die zurückliegenden 24 Stunden bestimmt werden, wobei insbesondere auch vorgesehen sein kann, dass nach diesem Intervall die ermittelte Kennzahl mit einer entsprechenden vorgegebenen Kennzahl verglichen wird und bei einer Abweichung ein Signal erzeugt wird. Es kann somit über einen langen Zeitraum eine zuverlässige Desinfektion gewährleistet werden.

Es kann auch vorgesehen sein, dass die Kennzahl während der Bestromung fortlaufend ermittelt wird, wobei nach Erreichen einer vorgegebenen Kennzahl die Bestromung der UV-LED gestoppt wird. Die Desinfektionseinrichtung kann somit energiesparend betrieben werden.

Insbesondere in diesem Zusammenhang kann vorgesehen sein, dass die Steuereinheit so eingerichtet ist, dass die mindestens eine UV-LED in vorgebbaren Intervallen automatisch mit einem Versorgungsstrom bestromt wird. Beispielsweise könnte vorgegeben werden, dass die UV-LED in einem gewissen Zeitfenster automatisch bis zum Erreichen der vorgegebenen Kennzahl betrieben wird. Wenn in diesem Zeitintervall dann die vorgegebene Kennzahl nicht erreicht wird, kann ein entsprechendes Signal erzeugt werden oder die Bestromung verlängert werden.

Wenn eine Unterbrechung der Bestromung von der Steuereinheit festgestellt wird, kann die Unterbrechung bei der Ermittlung der Kennzahl berücksichtigt werden.

Wie bereits oben ausgeführt, können die hinterlegten Daten eine Änderung der Abhängigkeit der zumindest einen Größe von der zurückliegenden Gesamtbestromungsdauer berücksichtigen, so dass die Degradation der UV-LED bei der Ermittlung der Kennzahl berücksichtigt wird.

Die hinterlegten Daten können nicht nur zur Ermittlung der Kennzahl, sondern auch zur Einstellung/Steuerung/Regelung der Versorgungsstromhöhe von der Steuereinheit benutzt werden. So kann beispielsweise vorgesehen sein, dass mit zunehmender Degradation der UV-LED die Versorgungsstromhöhe (also die Höhe des konstanten Gleichstroms beziehungsweise die Pulshöhe, Pulsbreite und/oder Pulswiederholfrequenz des gepulsten Gleichstroms) in den vorhandenen physikalischen Grenzen erhöht wird, so dass die UV-LED auch während der Degradation den gleichen Strahlungsfluss emittiert.

In den hinterlegten Daten kann auch ein Datensatz hinterlegt sein, der wiedergibt, bei Erreichen welcher Gesamtbetriebsdauer die UV-LED keinen ausreichenden Strahlungsfluss mehr emittieren kann. Dies kann beispielsweise der Fall sein, wenn die UV-LED in einem vorgegebenen Zeitintervall nicht mehr die ausreichende Bestrahlungsstärke zur Desinfektion einer Oberfläche bereitstellen kann.

Zusätzlich oder alternativ kann aber auch vorgesehen sein, dass die Versorgungsstromhöhe in Abhängigkeit einer in der Steuereinheit hinterlegbaren Raumgröße und/oder Raumgeometrie eines Raumes, in dem die Desinfektionseinrichtung betrieben wird, eingestellt wird. Die Desinfektionseinrichtung kann also für den jeweiligen Einsatzort individuell konfiguriert werden.

Wie bereits oben angedeutet, kann in den hinterlegten Daten eine Abhängigkeit zumindest einer Größe des von der mindestens einen UV-LED emittierten Lichts von einem die mindestens eine UV-LED versorgenden Versorgungsstrom für unterschiedliche Temperaturen wiedergegeben sein, wobei während des Bestromens eine Temperatur erfasst wird und die erfasste Temperatur bei der Bestimmung der Kennzahl berücksichtigt wird. Somit gibt die bestimmte Kennzahl die sie tatsächlich repräsentierende Größe genauer wieder.

In einer weiteren Ausgestaltung kann zusätzlich oder alternativ vorgesehen sein, dass in den hinterlegten Daten eine Abhängigkeit zumindest einer Größe des von der mindestens einen UV-LED emittierten Lichts von einem die mindestens eine UV-LED versorgenden Versorgungsstrom für unterschiedliche Temperaturen wiedergegeben ist, wobei während des Bestromens eine Temperatur erfasst wird und die Versorgungsstromhöhe unter Berücksichtigung der erfassten Temperatur während des Bestromens eingestellt wird. Es kann also auch vorgesehen sein, dass während des Bestromens die Versorgungsstromhöhe in Abhängigkeit einer erfassten Temperatur unter Rückgriff auf die hinterlegten Daten eingestellt wird. Die für den Einsatzort benötigte Strahlungsleistung kann somit genauer eingestellt werden.

Das Spektrum des von der UV-LED emittierten ultravioletten Lichts hat insbesondere eine Peak-Wellenlänge im Bereich zwischen 230 nm und 300 nm.

Die Desinfektionseinrichtung ist bevorzugt in der Art einer Leuchte und insbesondere bevorzugt in der Art einer Deckenleuchte ausgebildet. Dementsprechend weist die Desinfektionseinrichtung ein Gehäuse auf, in dem das die UV-LED aufweisende LED-Modul derart angeordnet ist, dass die emittierte ultraviolette Strahlung aus dem Gehäuse nach außen treten kann. Die Desinfektionseinrichtung ist also insbesondere zur Bestrahlung von Flächen eingerichtet, die außerhalb der Desinfektionseinrichtung angeordnet sind.

Zur Abführung der bei der Bestromung der UV-LED anfallenden Wärme ist ein Thermomanagement vorgesehen, welches passiv oder aktiv mittels einer Kühlung ausgebildet sein kann.

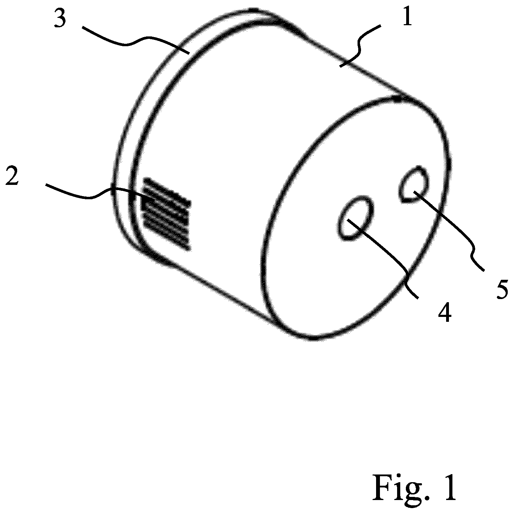

Die Erfindung sowie das technische Umfeld werden anhand der folgenden Zeichnung beispielhaft erläutert. Die Figuren zeigen schematisch
- Figur 1:: eine Perspektivansicht einer Desinfektionseinrichtung und
- Figur 2:: eine Schnittansicht durch die Desinfektionseinrichtung.

Die Desinfektionseinrichtung umfasst ein Gehäuse 1 mit Kühlrippen 2, mittels welchen Wärme von den in dem Gehäuse 1 angeordneten Komponenten abgeführt werden kann.

Das Gehäuse 1 weist auf der Oberseite einen Deckel 3 auf, mit welchem die Desinfektionseinrichtung an einer Decke befestigt werden kann. Auf der Unterseite weist das Gehäuse zwei Öffnungen 4, 5 auf. Durch die eine Öffnung 4 kann ultraviolettes Licht hindurchtreten (siehe weiter unten) und durch die andere Öffnung 5 kann ein Sensor betrieben werden.

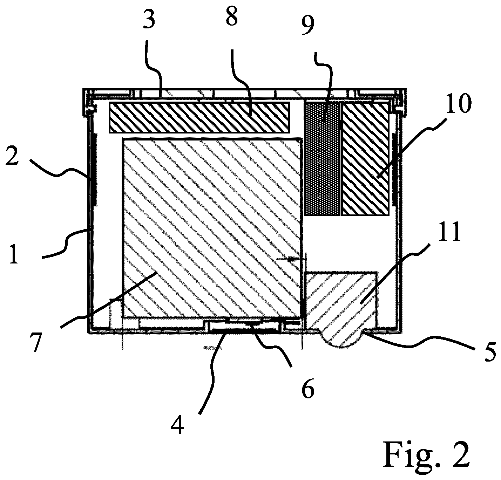

Wie insbesondere aus der Schnittansicht der Figur 2 hervorgeht, ist in dem Gehäuse 1 eine UV-LED 6 angeordnet, die ultraviolettes Licht mit einer Peak-Wellenlänge zwischen 230 nm und 300 nm emittiert. Das von der UV-LED 6 emittierte ultraviolette Licht tritt durch die Öffnung 6, welche durch eine für ultraviolettes Licht transparente Abdeckung verschlossen ist, aus der Desinfektionseinrichtung heraus. Die von dem ultravioletten Licht beaufschlagte Oberfläche kann somit desinfiziert werden.

Die UV-LED 6 ist Bestandteil eines LED-Moduls, welches auf einem Kühlkörper 7 montiert ist. Das LED-Modul ist mit einem LED-Treiber 8 für konstanten Gleichstrom verbunden, welcher wiederum mit einer Steuereinheit 9 verbunden ist. Die Steuereinheit 9 kann somit eine Versorgungstromhöhe des die UV-LED 6 versorgenden Versorgungsgleichstroms einstellen und erfassen.

Die Steuereinheit 9 ist zudem mit einer Kommunikationseinheit 10 verbunden. Über die Kommunikationseinheit 10 können Signale/Daten an eine übergeordnete Steuervorrichtung oder an ein Endgerät eines Benutzers übermittelt werden. Die Kommunikationseinheit 10 umfasst zudem einen Zeitsignale bereitstellenden Zeitgeber. Alternativ kann der Zeitgeber auch in der Steuereinheit 9 ausgebildet sein. Die Steuereinheit kann somit die Bestromungsdauer der UV-LED 6 erfassen.

Darüber hinaus weist die Desinfektionseinrichtung einen Sensor 11 zur Detektion eines Lebewesens und/oder einer Bewegung auf. Der Sensor 11 ist so verschaltet, dass bei einer Detektion eines Lebewesens und/oder einer Bewegung die Bestromung der UV-LED 6 unterbunden ist.

Die Steuereinheit 9 ist so eingerichtet, dass die UV-LED 6 durch Bestromung in vorgegebenen Intervallen eingeschaltet wird. Während der Bestromung wird die Versorgungsstromhöhe des Versorgungsstroms und die Bestromungsdauer auf Basis der von dem Zeitgeber vorgegebenen Zeitsignale erfasst.

In der Steuereinheit 9 sind zudem Daten hinterlegt, die zumindest eine Größe des von der mindestens einen UV-LED emittierten Lichts in Abhängigkeit der Versorgungsstromhöhe des die mindestens eine UV-LED versorgenden Versorgungsstroms wiedergeben.

Nach Beendigung der Bestromung oder in vorgegebenen Intervallen wird auf Basis der erfassten Versorgungsstromhöhe und der erfassten Bestromungsdauer unter Rückgriff auf die hinterlegten Daten eine Kennzahl ermittelt, die eine physikalische Größe des von der mindestens einen UV-LED emittierten Lichts wiedergibt. Beispielsweise gibt die Kennzahl die Strahlungsenergie des während des Bestromens emittierten ultravioletten Lichts wieder. Wenn auch die Anordnung und Geometrie der bestrahlten Fläche bekannt ist, lässt sich auf die Bestrahlungsstärke schließen. Aus der Kennzahl kann also geschlossen werden, ob eine Desinfektion der bestrahlten Oberfläche erfolgt ist.

Wenn die Kennzahl in einem vorgegebenen Intervall (beispielsweise 24 Stunden) von einer vorgegebenen Kennzahl für dieses Intervall abweicht, so kann ein (Warn)-Signal erzeugt werden, welches mittels der Kommunikationseinheit 10 an eine Leitstelle oder an ein Endgerät eines Benutzers übermittelt wird. In diesem Fall kann die Desinfektionseinrichtung überprüft werden beziehungsweise der Raum auf andere Weise desinfiziert werden.

### Bezugszeichenliste

- 1: Gehäuse
- 2: Kühlrippen
- 3: Deckel
- 4: Öffnung
- 5: Öffnung
- 6: UV-LED
- 7: Kühlkörper
- 8: LED Treiber
- 9: Steuereinheit
- 10: Kommunikationseinheit
- 11: Sensor

## Patentansprüche

1. Verfahren zum Betreiben einer mindestens eine UV-LED (6) umfassenden Desinfektionseinrichtung, umfassend zumindest die folgenden Schritte:
- Bestromen der mindestens einen UV-LED (6) mit einem Versorgungsstrom,
- Bereitstellen eines Zeitsignals,
- Betreiben der Desinfektionseinrichtung unter Verwendung des erfassten Zeitsignals,
- Hinterlegen von Daten in einer Steuereinheit der Desinfektionseinrichtung, wobei die Daten zumindest eine Größe des von der mindestens einen UV-LED (6) emittierten Lichts in Abhängigkeit eines die mindestens eine UV-LED (6) versorgenden Versorgungstroms wiedergeben.

2. Verfahren nach Anspruch 1, wobei die mindestens eine UV-LED (6) in vorgebaren Intervallen automatisch mit einem Versorgungsstrom bestromt wird.

3. Verfahren nach einem der vorhergehenden Ansprüche, wobei der Raum, in dem die Desinfektionseinrichtung betrieben wird, mittels eines Sensors (11) auf die Anwesenheit eines Lebewesens und/oder auf eine Bewegung überwacht wird, wobei bei der Detektion eines Lebewesens und/oder einer Bewegung die Bestromung verhindert wird.

4. Verfahren nach einem der vorhergehenden Ansprüche, wobei das Verfahren die folgenden Schritte umfasst:
- Erfassen einer Versorgungsstromhöhe des Versorgungsstroms,
- Erfassen einer Bestromungsdauer,
- Ermitteln einer Kennzahl des von der mindestens einen UV-LED (6) emittierten Lichts auf Basis der erfassten Versorgungsstromhöhe und der erfassten Bestromungsdauer unter Rückgriff auf die hinterlegten Daten.

5. Verfahren nach Anspruch 4, wobei die Kennzahl die Strahlungsenergie des während des Bestromens emittierten Lichts repräsentiert.

6. Verfahren nach Anspruch 4 oder 5, wobei die ermittelte Kennzahl mit einer vorgegebenen Kennzahl verglichen wird und bei einer Abweichung der ermittelten Kennzahl von der vorgegebenen Kennzahl ein Signal erzeugt wird.

7. Verfahren nach Anspruch 6, wobei die vorgegebene Kennzahl in Abhängigkeit einer Raumgröße und /oder einer Raumgeometrie eines Raumes, in dem die Desinfektionseinrichtung betrieben wird, einstellbar ist.

8. Verfahren nach einem der vorhergehenden Ansprüche 4 bis 7, wobei die Kennzahl jeweils nach einem Intervall für das zurückliegende Intervall bestimmt wird.

9. Verfahren nach einem der vorhergehenden Ansprüche 3 bis 8, wobei die hinterlegten Daten eine Änderung der Abhängigkeit der zumindest einen Größe von der zurückliegenden Gesamtbestromungsdauer berücksichtigen.

10. Verfahren nach einem der vorhergehenden Ansprüche 3 bis 9, wobei während des Bestromens die Versorgungsstromhöhe unter Rückgriff auf die hinterlegten Daten eingestellt wird.

11. Verfahren nach einem der vorhergehenden Ansprüche, wobei der Versorgungsstrom ein kontinuierlicher Gleichstrom ist.

12. Desinfektionseinrichtung, insbesondere eingerichtet zur Durchführung eines Verfahrens gemäß einem der vorstehenden Ansprüche, umfassend
- mindestens eine ultraviolettes Licht emittierende UV-LED (6) und
- eine Steuereinheit (9),
- einen Zeitgeber,
**dadurch gekennzeichnet, dass**
in der Steuereinheit (9) Daten hinterlegt sind, wobei die Daten zumindest eine Größe des von der mindestens einen UV-LED (6) emittierten Lichts in Abhängigkeit eines die mindestens eine UV-LED (6) versorgenden Versorgungstroms wiedergeben.

13. Desinfektionseinrichtung nach Anspruch 12, wobei die Desinfektionsreinrichtung mindestens einen Sensor (11) zur Erkennung eines Lebewesens und/oder einer Bewegung umfasst.

14. Desinfektionseinrichtung nach einem Anspruch 12 oder 13, wobei die Desinfektionseinrichtung einen Temperatursensor umfasst.

## Claims

1. Method for operating a disinfection device comprising at least one UV-LED (6), comprising at least the following steps:
- powering the at least one UV LED (6) with a supply current,
- providing a time signal,
- operating the disinfecting device using the acquired time signal,
- storing data in a control unit of the disinfection device, wherein the data reproduces at least one quantity of the light emitted by the at least one UV LED (6) as a function of a supply current supplying the at least one UV LED (6).

2. Method according to Claim 1, wherein the at least one UV LED (6) is automatically powered with a supply current at predeterminable intervals.

3. Method according to any of the preceding claims, wherein the room in which the disinfection device is operated is monitored by means of a sensor (11) for the presence of an organism and/or for a movement, wherein the supply of power is prevented upon the detection of an organism and/or a movement.

4. Method according to any of the preceding claims, wherein the method comprises the following steps:
- detecting a supply current level of the supply current,
- recording a power supply duration,
- determining a characteristic parameter of the light emitted by the at least one UV LED (6) on the basis of the detected supply current level and the recorded power supply duration by using the stored data.

5. Method according to Claim 4, wherein the characteristic parameter represents the radiation energy of the light emitted while power is being supplied.

6. Method according to Claim 4 or 5, wherein the determined characteristic parameter is compared with a predefined characteristic parameter and a signal is generated in the event of a deviation of the determined characteristic parameter from the predefined characteristic parameter.

7. Method according to Claim 6, wherein the predetermined characteristic parameter is adjustable depending on a room size and/or room geometry of a room in which the disinfection device is operated.

8. Method according to any of the preceding Claims 4 to 7, wherein the characteristic parameter is determined after each interval for the previous interval.

9. Method according to any of the preceding Claims 3 to 8, wherein the stored data take into account a change in the dependence of at least one quantity on the previous total power supply duration.

10. Method according to any of the preceding claims 3 to 9, wherein the supply current level while power is being supplied is set using the stored data.

11. Method according to any of the preceding claims, wherein the supply current is a continuous DC current.

12. Disinfection device, in particular configured for carrying out a method according to any of the preceding claims, comprising
- at least one ultraviolet light-emitting UV LED (6) and
- a control unit (9),
- a timer,
**characterized in that**
the control unit (9) contains stored data, wherein the data reproduces at least one quantity of the light emitted by the at least one UV LED (6) as a function of a supply current supplying the at least one UV LED (6).

13. Disinfection device according to Claim 12, wherein the disinfection device comprises at least one sensor (11) for detecting an organism and/or a movement.

14. Disinfection device according to either of Claims 12 or 13, wherein the disinfection device comprises a temperature sensor.

## Revendications

1. Procédé destiné à faire fonctionner un dispositif de désinfection comprenant au moins une diode électroluminescente à ultraviolet (DEL-UV) (6), comprenant les étapes suivantes :
- alimentation en courant d'au moins une DEL-UV(6) avec un courant d'alimentation,
- préparation d'un signal de temps,
- fonctionnement du dispositif de désinfection en utilisant le signal de temps saisi,
- mémorisation de données dans une unité de commande du dispositif de désinfection, sachant que les données restituent au moins une valeur de la lumière émise par au moins une DEL-UV (6) en fonction d'au moins un courant d'alimentation alimentant au moins une DEL-UV (6).

2. Procédé selon la revendication 1, sachant qu'au moins une DEL-UV (6) est automatiquement alimentée en courant à des intervalles prédéfinissables avec un courant d'alimentation.

3. Procédé selon l'une quelconque des revendications précédentes, sachant que l'espace dans lequel le dispositif de désinfection est utilisé, est surveillé au moyen d'un capteur (11) en ce qui concerne la présence d'un être vivant et/ou d'un mouvement, sachant que l'alimentation en courant est empêchée lors de la détection d'un être vivant et/ou d'un mouvement.

4. Procédé selon l'une quelconque des revendications précédentes, sachant que le procédé comprend les étapes suivantes :
- saisie d'une intensité de courant d'alimentation du courant d'alimentation,
- saisie d'une durée d'alimentation en courant,
- détermination d'un indice de la lumière émise par au moins une DEL-UV (6) sur la base de l'intensité de courant d'alimentation saisie et de la durée d'alimentation en courant saisie en tenant compte des données mémorisées.

5. Procédé selon la revendication 4, sachant que l'indice représente l'énergie de rayonnement de la lumière émise pendant l'alimentation en courant.

6. Procédé selon la revendication 4 ou 5, sachant que l'indice déterminé est comparé à un indice prédéfini et qu'un signal est généré en cas d'écart de l'indice déterminé par rapport à l'indice prédéfini.

7. Procédé selon la revendication 6, sachant que l'indice prédéfini peut être réglé en fonction d'une grandeur d'espace et/ou d'une géométrie spatiale d'un espace dans lequel le dispositif de désinfection est utilisé.

8. Procédé selon l'une quelconque des revendications précédentes 4 à 7, sachant que l'indice est respectivement déterminé selon un intervalle pour l'intervalle antérieur.

9. Procédé selon l'une quelconque des revendications précédentes 3 à 8, sachant que les données mémorisées prennent en considération une modification d'au moins une valeur en fonction de la durée totale d'alimentation en courant antérieure.

10. Procédé selon l'une quelconque des revendications précédentes 3 à 9, sachant que pendant l'alimentation en courant, l'intensité du courant d'alimentation est réglée en tenant compte des données mémorisées.

11. Procédé selon l'une quelconque des revendications précédentes, sachant que le courant d'alimentation est un courant continu.

12. Dispositif de désinfection, agencé en particulier pour exécuter un procédé selon l'une quelconque des revendications précédentes, comprenant :
- au moins une diode électroluminescente à ultraviolet (DEL-UV) émettant une lumière ultraviolette(6) et
- une unité de commande (9),
- un compteur de temps,
**caractérisé en ce que**
des données sont mémorisées dans l'unité de commande (9), sachant que les données restituent au moins une valeur de la lumière émise par au moins une DEL-UV (6) en fonction d'un courant d'alimentation alimentant au moins une DEL-UV (6).

13. Dispositif de désinfection selon la revendication 12, sachant que le dispositif de désinfection comprend au moins un capteur (11) pour identifier un être vivant et/ou un mouvement.

14. Dispositif de désinfection selon l'une quelconque des revendications 12 ou 13, sachant que le dispositif de désinfection comprend un capteur de température.
